(19) Europäisches Patentamt

European Patent Office

Office européen des brevets

(11) Veröffentlichungsnummer: **0 304 499 B1**

## (12) EUROPÄISCHE PATENTSCHRIFT

(45) Veröffentlichungstag der Patentschrift: **04.03.92**

(51) Int. Cl.⁵: **C07D 323/06**

(21) Anmeldenummer: **87112197.6**

(22) Anmeldetag: **22.08.87**

Die Akte enthält technische Angaben, die nach dem Eingang der Anmeldung eingereicht wurden und die nicht in dieser Patentschrift enthalten sind.

(54) **Verfahren zur Isolierung von Trioxan aus wässrigen Trioxanlösungen durch destillative Trennung.**

(43) Veröffentlichungstag der Anmeldung:
**01.03.89 Patentblatt  89/09**

(45) Bekanntmachung des Hinweises auf die Patenterteilung:
**04.03.92 Patentblatt  92/10**

(84) Benannte Vertragsstaaten:
**BE CH DE FR GB IT LI NL SE**

(56) Entgegenhaltungen:

**CHEMICAL ABSTRACTS, Band 102, 1985, Seite 115, Zusammenfassung Nr. 115532s, Columbus, Ohio, US; & JP-A-59 186 975 (MITSUI PETROCHMICAL INDUSTRIES LTD) 23-10-1984**

**Römpps Chemielexikon ISBN 3-440-04510-2 Seiten 908 und 909**

(73) Patentinhaber: **Küppenbender, Herbert Lenzhahner Weg 36 W-6272 Niedernhausen(DE)**

Patentinhaber: **Reis, Helmut Köhlersweg 15 W-8751 Hofstetten(DE)**

(72) Erfinder: **Küppenbender, Herbert Lenzhahner Weg 36 W-6272 Niedernhausen(DE)**
Erfinder: **Reis, Helmut Köhlersweg 15 W-8751 Hofstetten(DE)**

(74) Vertreter: **Bartsch, Elisabeth, Dr. Patentanwälte Lotterhos & Partner Lichtensteinstrasse 3 W-6000 Frankfurt am Main 1(DE)**

**Beschreibung**

Trioxan gewinnt zunehmend an Bedeutung, z.B. als Zwischenprodukt für die Produktion von Polyacetalen. Es wird durch Trimerisierung von Formaldehyd erhalten, wobei gewöhnlich von einer wäßrigen Formalinlösung mit einem Formaldehydgehalt im Bereich von etwa 30 bis 70 % ausgegangen wird, die in einem Trioxanreaktor in Gegenwart eines Säurekatalysators zum Sieden erhitzt wird. Das hierbei gebildete Trioxan wird aus dem Trioxanreaktor in Form eines im wesentlichen aus Trioxan, Wasser und Formaldehyd bestehenden Dampfgemisches, des sogenannten Synthesedampfes, abdestilliert. Der Säurekatalysator bleibt im Reaktor zurück. Der so erhaltene Synthesedampf, der einen Trioxangehalt von etwa 12 bis 16 Gew.% aufweist, wird gewöhnlich zur Erhöhung der Trioxankonzentration durch eine, dem Trioxanreaktor nachgeschaltete Rektifizierungskolonne geleitet, wobei vorwiegend Wasser und Formaldehyd in den Kolonnensumpf gehen, während am Kolonnenkopf eine Trioxan-Wasser-Formaldehyd-Mischung erhalten wird, die Trioxan, Wasser und Formaldehyd im Verhältnis von etwa 35 : 48 : 17 enthält.

Da Trioxan mit 30 Gew.% Wasser bekanntlich ein Azeotrop bildet, das einer Isolierung des Trioxans aus wäßrigen Lösungen auf dem üblichen Destillationsweg entgegensteht, wird zur Abtrennung des Wassers und zur Isolierung des Trioxans in reiner Form die am Kolonnenkopf erhaltene wäßrige Trioxanlösung gewöhnlich einer Extraktionsbehandlung mit einem inerten, mit Wasser nicht mischbaren Lösungsmittel, vorzugsweise den Chlorkohlenwasserstoffen: Methylenchlorid oder Äthylenchlorid, oder Benzol, unterworfen.

Die mit der Extraktionsbehandlung verbundenen Nachteile bestehen nicht nur darin, daß die Abtrennung des Trioxans vom Extraktionsmittel und dessen Reinigung vor der Wiederverwendung zusätzliche Arbeitsgänge erfordern. Sie bestehen vor allem darin, daß es sich beim Arbeiten mit organischen Lösungsmitteln gewöhnlich nicht vermeiden läßt, daß Lösungsmittel in die Abwässer gelangen und in die Atmosphäre entweichen, in der die organischen Lösungsmittel und insbesondere die Chlorkohlenwasserstoffe im Zusammenwirken mit UV-Strahlung zu einer starken Umweltbelastung werden können.

Aus CA 102: 115 532s (JP-A 59-186 975) ist bereits bekannt, zur Isolierung von Trioxan wäßrige, Formaldehyd enthaltende Trioxanlösungen mit Inertgasen in überkritischen Zustand einer Extraktionsbehandlung zu unterwerfen. Die Trioxankonzentrationen der mit diesem Verfahren erhältlichen wäßrigen Lösungen erfordern aber eine weitere Extraktion mit organischen Lösungsmitteln zur Isolierung von hochkonzentriertem Trioxan.

Es wurde gefunden, daß sich die bei der Isolierung von reinem Trioxan aus wäßrigen Trioxanlösungen bislang üblichen Lösungsmittelextraktionen sowie die damit verbundenen Nachteile in überraschend einfacher Weise umgehen lassen, wenn man

das beim Erhitzen der wäßrigen Trioxanlösung zum Sieden aus dem Verdampfer abdestillierende Dampfgemisch,

in Stufe 1 durch einen, unmittelbar nach dem Verdampfer angeordneten Partialkondensator leitet, in dem das Dampfgemisch in Gegenwart eines Inertgasstromes,

der entweder in den Verdampfer oder in den unteren Teil des Partialkondensators eingeführt wird,

einer fraktionerten Kondensation im Temperaturbereich von 58 - 64 °C unterworfen wird,

wobei der Wasseranteil des Dampfgemisches kondensiert und abgetrennt wird, und

den so erhaltenen Trioxananteil des Dampfgemisches zusammen mit dem Inertgasstrom

in Stufe 2 durch einen unmittelbar nach dem Partialkondensator angeordneten Absorber oder Kondensator leitet, in dem das Trioxan und gegebenenfalls vorhandenes Rest-Wasser kondensiert und von dem Inertgasstrom abgetrennt wird.

In dem Partialkondensator der Stufe 1, der mit entsprechend temperiertem Kühlmedium (zweckmäßig Wasser) beschickt wird, wird der mit Trioxan-enthaltendem Wasserdampf beladene Inertgasstrom, der beim Eintritt in den Partialkondensator eine Temperatur von etwa 92 °C aufweist, auf eine Temperatur im Bereich von 58 bis 64 °C, insbesondere von 60 bis 62 °C, gekühlt. Hierbei wird der Wasseranteil des Dampfgemisches partiell kondensiert und abgetrennt. Da das so erhaltene Kondensat praktisch kein Trioxan mehr enthält, ist es möglich, mit der partiellen Kondensation in Gegenwart des Inertgasstromes gemäß Erfindung den gesamten Wasseranteil aus dem Trioxan-enthaltenden Dampfgemisch selektiv zu entfernen. Aus praktischen Gründen wird der Wasseranteil nur bis auf etwa 10 Gew.% entfernt.

Der so erhaltene, vom Wasser praktisch befreite Trioxananteil des Dampfgemisches wird in Stufe 2 zusammen mit dem Inertgasstrom durch einen dem Partialkondensator unmittelbar nachgeordneten Absorber oder Kondensator geleitet, dort kondensiert und vom Inertgasstrom abgetrennt.

Das so erhaltene wäßrige Trioxankondensat mit einem Trioxangehalt von mindestens 90 Gew.% läßt sich ohne Schwierigkeiten in an sich bekannter Weise, z.B. durch Kristallisation, auf wasserfreies, reines Trioxan aufarbeiten.

Anstelle des Partialkondensators kann in Stufe 1 auch ein Dephlegmator oder eine Destillationsko

Ionne verwendet werden.

Die Zusammensetzung des Inertgases ist nicht kritisch. Einige Beispiele hierfür sind: Stickstoff, Luft, sauerstoffarme Luft (Sauerstoffgehalt etwa 10 bis 12 Vol.%), Kohlendioxid, Edelgas, etc..

Der Inertgasstrom wird zweckmäßig im Kreislauf geführt, um Verluste an mitgerissenem Trioxan zu vermeiden. Er kann in den Verdampfer oder in den unteren Teil des Partialkondensators oder aber auch in die Verbindungsleitung zwischen Verdampfer und Partialkondensator eingeleitet werden. Vorzugsweise wird der Inertgasstrom in den Bodenteil des Verdampfers eingeleitet, um eine gute Verteilung des Inertgases in der im Verdampfer enthaltenen Lösung zu erzielen. Zu diesem Zweck kann der Verdampfer mit Einrichtungen, wie Füllkörpern, tragenden Verteilerboden, z.B. Sieb-, Düsen-, Ventil-, Glockenböden, ausgestattet werden, die die bereits mit der Einleitung des Inertgasstromes in die Verdampferlösung erzeugte Durchmischung verstärken.

Die Isolierung des Trioxans in Form von etwa 90 Gew.%igen wäßrigen Trioxankonzentraten durch das destillative Trennverfahren gemäß Erfindung kann auf wäßrige Trioxanlösungen unabhängig von deren Trioxangehalt angewandt werden, sie kann diskontinuierlich oder kontinuierlich durchgeführt werden. Wird das destillative Trennverfahren der Erfindung diskontinuierlich durchgeführt, kann der in Stufe 1 durch partielle Kondensation abgeschiedene Wasseranteil des Dampfgemisches in den Verdampfer zur Aufrechterhaltung der notwendigen Flüssigkeitsmenge zurückgeleitet werden. Wird dagegen dem Verdampfer ständig neue Flüssigkeit zugeführt, wie das beim kontinuierlichen Verfahren der Fall ist, wird der durch partielle Kondensation abgeschiedene Wasseranteil aus dem System herausgeführt.

Das destillative Trennverfahren der Erfindung unter Isolierung von Trioxan in Form eines etwa 90 Gew.%igen wäßrigen Trioxankonzentrats läßt sich auch auf Trioxanlösungen anwenden, die außer Wasser noch Formaldehyd enthalten. Hierbei wird der Formaldehydanteil des Dampfgemisches, das in Stufe 1 zusammen mit dem Inertgasstrom durch den Partialkondensator hindurchgeführt und auf eine Temperatur im im Bereich von 58 bis 64°C, vorzugsweise von 60 bis 62°C, gekühlt wird, zusammen mit dem Wasseranteil des Dampfgemisches kondensiert und abgetrennt. Der so erhaltene wasserarme Trioxananteil, der dann der Kondensation in Gegenwart des Intergasstromes in Stufe 2 unterworfen wird, weist praktisch keinen Formaldehydgeruch mehr auf.

Dieser Anwendungsbereich des Trioxanisolierungsverfahrens der Erfindung ist besonders vorteilhaft. Das Trioxan wird bekanntlich bei den üblichen Syntheseverfahren, bei denen der aus dem Trioxanreaktor abdestillierende Synthesedampf zur Erhöhung der Trioxankonzentration durch eine Rektifizierungskolonne geleitet wird, am Kolonnenkopf in Form einer wäßrigen Trioxan-Formaldehyd-Lösung erhalten, in der Trioxan, Wasser und Formaldehyd im Verhältnis von etwa 35 : 48 : 17 vorliegen. Aus derartigen Lösungen, aus denen das Trioxan bislang nur durch Anwendung der bekannten Lösungsmittelextraktion isoliert werden konnte, kann nun das Trioxan allein durch das destillative Trennverfahren der Erfindung isoliert werden.

Das destillative Trennverfahren gemäß Erfindung kann auch auf eine saure, Trioxan, Wasser und Formaldehyd enthaltende Lösung angewandt werden, die beim Erhitzen saurer, etwa 30 bis 70 Gew.%iger, vorzugsweise etwa 50 bis 65 Gew.%iger Formalinlösungen mit einem pH-Wert, der dem einer 0,5 bis 25 Gew.%igen Schwefelsäure entspricht, erhalten wird. In diesen Lösungen bildet sich beim Erhitzen in situ weiteres Trioxan, das gewöhnlich aus dem Reaktionsgefäß zusammen mit Wasser und Formaldehyd abdestilliert wird. Das aus dem Reaktor abdestillierende Dampfgemisch, der sogenannte Synthesedampf, enthält keine Säure.

Bei Anwendung des Trennverfahrens der Erfindung auf derartige Lösungen wird das Inertgas in das Reaktionsgefäß - vorzugsweise in dessen Bodenteil - eingeleitet und zusammen mit dem aus dem Reaktionsgefäß abdestillierenden Dampfgemisch, das eine Trioxankonzentration von etwa 55 Gew.% und eine Temperatur von etwa 92°C aufweist, durch den Partialkondensator der Stufe 1 hindurchgeführt. Hierbei wird der Formaldehydanteil des Dampfgemisches beim Abkühlen auf eine Temperatur im Bereich von 58 bis 64°C, vorzugsweise von 60 bis 62°C, zusammen mit dem Wasseranteil des Dampfgemisches kondensiert und abgetrennt und/oder in den Reaktor zur Aufrechterhaltung der notwendigen Flüssigkeitsmenge zurückgeleitet, je nachdem ob das Verfahren gemäß Erfindung kontinuierlich oder diskontinuierlich durchgeführt wird.

Der so erhaltene Trioxananteil des Dampfgemisches, der zusammen mit dem restlichen Wasseranteil und dem Inertgasstrom in Stufe 2 der Kondensation unterworfen wird, weist praktisch keinen Formaldehydgeruch mehr auf. Das nach der Kondensation erhaltene wäßrige Trioxankonzentrat weist einen Trioxangehalt von mehr als 90 Gew.% auf und läßt sich ohne Schwierigkeiten, ohne Anwendung von Lösungsmittelextraktion auf wasserfreies, reines Trioxan aufarbeiten.

Bei Anwendung des destillativen Trennverfahrens der Erfindung auf saure, Trioxan-enthaltende Formalinlösungen, die beim Erhitzen weiteres Trioxan in situ bilden, kann das Inertgas auch zusammen mit gasförmigem Formaldehyd in den Trioxanreaktor eingeleitet werden. Der gasförmige Formaldehyd beeinflußt den Verfahrensablauf des Trennverfahrens der Erfindung nicht, da er in der heißen sauren Reaktorflüssigkeit sofort in Trioxan übergeführt wird, das in Form des Synthesedampfes aus dem Reaktorgefäß abdestilliert und zusammen mit dem Inertgasstrom durch die weiteren Kondensationsstufen 1 und 2 des Trennverfahrens der Erfindung geführt wird. Die Anwendung von Formaldehyd enthaltendem Inertgas ermöglicht in besonders einfacher Weise mit dem Verfahren der Erfindung kontinuierlich Trioxankonzentrate mit einem Trioxangehalt von mehr als 90 % herzustellen.

Als Formaldehyd enthaltender Inertgasstrom kann auch ein aus der katalytischen Methanoloxydation, z.B. dem Formox-Verfahren oder dem Silberkontaktverfahren, stammender Formaldehydgasstrom verwendet werden. In den Fällen macht das inerte Begleitgas des Formaldehyds die gesonderte Inertgaszuführung überflüssig.

Das Verhältnis von Inertgas zu Flüssigkeitsdampf ist nicht kritisch. Es wird entweder durch das Trioxanherstellungsverfahren oder durch das Erreichen der Gassättigungsgrenze, des Taupunktes, der druck-und temperaturabhängig ist, bestimmt. Geht man bei Verfahrensdurchführung von einer wäßrigen Trioxanlösung aus, die verdampft wird, so führt man dem Dampfgemisch nur so viel Energie zu, daß der Taupunkt nicht unterschritten wird. Bei einer Beladung des Inertgases mit den Trioxan und Wasser enthaltenden Dampfgemischen bis nahe an den Taupunkt sind die besten Ergebnisse zu erzielen,und der apparative Aufwand ist am geringsten.

Mit dem Trennverfahren der Erfindung kann aus den Trioxan, Wasser und gegebenenfalls Formaldehyd enthaltenden Dampfgemischen theoretisch der gesamte Wasseranteil durch die partielle Kondensation im Dephlegmator entfernt werden. Da aber der Wasserabscheidungsgrad und die Kühlflächengröße im Dephlegmator asymptotisch verlaufen, würde die 100 %ige Entfernung des Wasseranteils eine unendlich große Kühlfläche im Dephlegmator erfordern. Infolgedessen wird aus wirtschaftlichen Gründen die Entfernung des Wasseranteils im Dephlegmator nur so weit geführt, daß die dann noch verbleibenden Restanteile an Wasser die weitere Aufarbeitung des Trioxans auf reines Produkt nicht stören. Dies ist der Fall, wenn der Wassergehalt des im Absorber oder Kondensator in Gegenwart eines Inertgases kondensierten Trioxans nicht mehr als 10 Gew.% beträgt.

Die Frage, ob in Stufe 2 ein Absorber oder ein Kondensator eingesetzt wird, hängt von dem Verhältnis von Dampfmischung zu Inertgas ab. Bei hohem Inertgasanteil wird ein Absorber, bei geringerem Inertgasanteil ein Kondensator benutzt.

Das destillative Trennverfahren der Erfindung soll durch die folgenden Beispiele näher erläutert werden.

Beispiel 1

In einem Verdampfer erhitzte man 1.400 g einer wäßrigen Trioxanlösung mit einem Trioxangehalt von 50 Gew.% unter Hindurchleiten eines Stickstoffstromes, der in den unteren Teil des Verdampfers eingeleitet und im Kreislauf geführt wurde, zum Sieden und führte das aus dem Verdampfer absdestillierende Dampfgemisch zusammen mit dem Stickstoffstrom in einen, dem Verdampfer unmittelbar nachgeordneten Dephlegmator, in dem der Wasseranteil des Dampfgemisches im Temperaturbereich von 60 bis 62 °C partiell kondensiert und anschließend in den Verdampfer zurückgeleitet wurde.

Der so erhaltene, vom Wasser weitgehend befreite Trioxananteil des Dampfgemisches wurde dann zusammen mit dem Stickstoffstrom in einen dem Dephlegmator unmittelbar nachgeordneten Kondensator geleitet und dort in Gegenwart des Stickstoffstromes kondensiert und in ein Auffanggefäß geleitet, während der Stickstoffstrom in den Verdampfer zurückgeführt wurde.

Es wurden 715 g Trioxan mit einem Wassergehalt von 9,0 Gew.% erhalten. Die Ausbeute betrug 93 %. Der Stickstoff wurde mit einer Geschwindigkeit von 60 l/Std. zirkulieren gelassen.

Die Temperatur im Verdampfer betrug 92 °C, sie stieg schlagartig auf 100 °C an, nachdem das gesamte Trioxan abdestilliert war.

Die gleichen Ergebnisse wurden erzielt, wenn der aus dem Kondensator austretende Stickstoffstrom statt in den Verdampfer in die Verbindungsleitung zwischen Verdampfer und Dephlegmator zurückgeleitet wurde.

Beispiel 2

Es wurde wie in Beispiel 1 verfahren, jedoch mit der Ausnahme, daß dem Verdampfer kontinuierlich eine wäßrige Trioxanlösung mit einem Trioxangehalt von 50 Gew.% in einer Menge von 0,47 l/Std. zugeführt und das im Dephlegmator kondensierte Wasser nicht in den Verdampfer zurückgeführt, sondern aus dem System herausgeführt wurde.

Die im Kondensator abgeschiedene Trioxanmenge betrug 705 g/Std.; das Trioxan hatte einen Wassergehalt von 8,3 Gew.%.

Beispiel 3

Anstelle der wäßrigen Trioxanlösung wurden 1 l einer zur Herstellung von Trioxan verwendeten 64 %igen Formalinlösung, die 8 Gew.% Schwefelsäure und 0,8 Gew.% Phosphorsäure als Katalysator enthielt, unter Einleiten eines Stickstoffstromes zum Sieden erhitzt. Das dabei abdestillierende Dampfgemisch, der Synthesedampf, der außer Wasser und Trioxan noch Formaldehyd enthielt, wurde zusammen mit dem Stickstoffstrom durch einen dem Trioxanreaktor unmittelbar nachgeordneten Dephlegmator,in dem der Gasstrom auf eine Temperatur im Bereich von 60 bis 62°C gekühlt wurde, hindurchgeleitet, wobei durch partielle Kondensation der Wasseranteil zusammen mit dem Formaldehyd abgeschieden und aus dem System herausgeführt wurde.

Der so erhaltene Trioxananteil des Synthesedampfes wurde dann zusammen mit dem Stickstoffstrom in einen dem Dephlegmator unmittelbar nachgeordneten Kondensator geleitet und dort in Gegenwart des Stickstoffstromes kondensiert und in den Trioxanabscheider geleitet, während der Stickstoffstrom in den Trioxanreaktor zurückgeführt wurde. Der Stickstoff wurde mit einer Geschwindigkeit von 60 l/Std. zirkulieren gelassen.

Es wurden 520 g Trioxan mit einem Wassergehalt von 9,2 Gew.% erhalten.

Beispiel 4

Es wurde wie in Beispiel 3 verfahren, jedoch mit der Ausnahme, daß man dem Trioxanreaktor kontinuierlich aus einem Druckverdampfer gasförmigen Formaldehyd, der in den zirkulierenden Stickstoffstrom eingeleitet wurde, zuführte.

Die im Kondensator in Gegenwart des Stickstoffgases abgeschiedene Trioxanmenge betrug 832 g/Std. und hatte einen Wassergehalt von 6,9 Gew.%.

**Patentansprüche**

1. Verfahren zur Isolierung von Trioxan aus wäßrigen Trioxanlösungen durch destillative Trennung ohne Verwendung organischer Lösungsmittel, dadurch gekennzeichnet, daß man
   das beim Erhitzen der wäßrigen Trioxanlösung zum Sieden aus dem Verdampfer abdestillierende Dampfgemisch,
   in Stufe 1 durch einen, unmittelbar nach dem Verdampfer angeordneten Partialkondensator leitet, in dem das Dampfgemisch in Gegenwart eines Inertgasstromes,
   der entweder in den Verdampfer oder in den unteren Teil des Partialkondensators eingeführt wird,
   einer fraktionerten Kondensation im Temperaturbereich von 58 - 64°C unterworfen wird,
   wobei der Wasseranteil des Dampfgemisches kondensiert und abgetrennt wird, und
   den so erhaltenen Trioxananteil des Dampfgemisches zusammen mit dem Inertgasstrom
in Stufe 2 durch einen unmittelbar nach dem Partialkondensator angeordneten Absorber oder Kondensator leitet, in dem das Trioxan und gegebenenfalls vorhandenes Rest-Wasser kondensiert und von dem Inertgasstrom abgetrennt wird.

2. Verfahren nach Anspruch 1, dadurch gekennzeichnet, daß man den Inertgasstrom in den unteren Teil des mit Einrichtungen zum Dispergieren des Inertgasstromes ausgestatteten Verdampfers oder in den unteren Teil des Partialkondensators einleitet.

3. Verfahren nach Anspruch 1 oder 2, dadurch gekennzeichnet, daß man den Inertgasstrom im Kreislauf führt.

4. Verfahren nach Anspruch 1, dadurch gekennzeichnet, das anstelle des Partialkondensators ein Dephlegmator oder eine Destillationskolonne verwendet wird.

5. Verfahren nach einem der Ansprüche 1 bis 3, dadurch gekennzeichnet, daß man als Inertgas Stickstoff, Luft oder sauerstoffarme Luft, Kohlendioxid oder Edelgas benutzt.

6. Verfahren nach einem der Ansprüche 1 bis 5, dadurch gekennzeichnet, daß man der destillativen Trennung eine azeotrope Trioxan-Wassermischung oder eine wäßrige Trioxan-Formaldehyd-Lösung unterwirft.

7. Verfahren nach einem der Ansprüche 1 bis 5, dadurch gekennzeichnet, daß man der destillativen Trennung eine saure, Trioxan, Wasser und Formaldehyd enthaltende Mischung unterwirft.

8. Verfahren nach einem der Ansprüche 1 bis 5, dadurch gekennzeichnet, daß man der destillativen Trennung eine saure, Trioxan, Wasser und Formaldehyd enthaltende Mischung unterwirft, und das destillative Trennverfahren in Gegenwart eines Formaldehyd enthaltendem Inertgasstromes durchführt.

9. Verfahren nach einem der Ansprüche 1 bis 4, dadurch gekennzeichnet, daß man der destillativen Trennung eine saure, Trioxan, Wasser und Formaldehyd enthaltende Mischung unterwirft, und als Formaldehyd enthaltenden Inertgasstrom einen von der katalytischen Methanoloxydation stammenden Formaldehydgasstrom verwendet.

10. Verfahren nach einem der Ansprüche 1, 6 - 9, dadurch gekennzeichnet, daß man die destillative Trennung diskontinuierlich durchführt, wobei der im Partialkondensator kondensierte Wasseranteil des aus dem Verdampfer abdestillierenden Dampfgemisches in das System zurückgeleitet wird.

11. Verfahren nach einem der Ansprüche 1, 6 und 7, dadurch gekennzeichnet, daß man die destillative Trennung kontinuierlich durchführt, wobei der im Partialkondensator kondensierte Wasseranteil des aus dem Verdampfer abdestillierenden Dampfgemisches aus dem System entfernt wird.

## Claims

1. A method of isolating trioxane from aqueous trioxane solutions by distillative separation without use of organic solvents,
characterised in that
the vapour mixture distilled off from the evaporator when the aqueous trioxane solution is heated to boiling-point is conveyed in stage 1 through a partial condenser which is disposed immediately behind the evaporator and in which the vapour mixture, in the presence of a stream of inert gas,
which is introduced either into the evaporator or into the bottom part of the partial condenser is subjected to fractional condensation in the temperature range from 58 - 64 °C,
when the water content of the vapour mixture condenses and is separated, and
in stage 2 the resulting trioxane component of the vapour mixture is conveyed together with the stream of inert gas through an absorber or condenser which is disposed immediately behind the partial condenser and in which the trioxane and any remaining water is condensed and separated from the stream of inert gas.

2. A method according to claim 1, characterised in that the stream of inert gas is introduced into the bottom part of the evaporator, which is equipped with means for dispersing the stream of inert gas, or is introduced into the bottom part of the partial condenser.

3. A method according to claim 1 or 2, characterised in that the stream of inert gas is conveyed in a circuit.

4. A method according to claim 1, characterised in that instead of the partial condenser, a dephlegmator or a distillation column is used.

5. A method according to any of claims 1 - 3, characterised in that the inert gas is nitrogen or air or oxygen-depleted air or carbon dioxide or a noble gas.

6. A method according to any of claims 1 - 5, characterised in that an azeotropic mixture of trioxane and water or an aqueous trioxane-formaldehyde solution is separated by distillation.

7. A method according to any of claims 1 - 5, characterised in that an acid mixture containing trioxane, water and formaldehyde is separated by distillation.

8. A method according to any of claims 1 - 5, characterised in that an acid mixture containing trioxane, water and formaldehyde is separated by distillation and the distillative separation process is carried out in the presence of a stream of inert gas containing formaldehyde.

9. A method according to any of claims 1 - 4, characterised in that an acid mixture containing trioxane, water and formaldehyde is separated by distillation and the stream of inert gas containing formaldehyde is obtained by catalytic oxidation of methanol.

10. A method according to claim 1 or claims 6 - 9, characterised in that separation by distillation is carried out discontinuously and the water content of the vapour mixture distilled off from the evaporator and condensed in the partial condenser is returned to the system.

11. A method according to claim 1 or claims 6 and 7, characterised in that separation by distillation is carried out continuously and the water content of the vapour mixture distilled off from the evaporator and condensed in the partial condenser is returned to the system.

## Revendications

1. Procédé d'isolement de trioxanne de solutions aqueuses de trioxanne par séparation par distillation sans utilisation de solvants organiques, caractérisé en ce que, lors du chauffage à ébulliton de la solution aqueuse de trioxanne,
à l'étape 1, on dirige le mélange de vapeur s'évacuant par distillation de l'évaporateur dans un condenseur à fractionnement disposé directement en aval de l'évaporateur et dans lequel le mélange de vapeur est soumis à une condensation fractionnée, dans la plage de température de 58-64 °C, en présence d'un courant de gaz inerte qui est introduit soit dans l'évaporateur, soit dans la partie inférieure du condenseur à fractionnement, le teneur en eau du mélange de vapeur étant condensée et séparée et

à l'étape 2, on dirige la fraction ainsi obtenue de trioxanne du mélange de vapeur avec le courant de gaz inerte dans un absorbeur ou un condenseur disposé directement en aval du condenseur à fractionnement et dans lequel le trioxanne et l'eau résiduelle éventuellement présente sont condensés et séparés du courant de gaz inerte.

2.  Procédé selon la revendication 1, caractérisé en ce que l'on introduit le courant de gaz inerte dans la partie inférieure de l'évaporateur équipé de dispositifs de dispersion du courant de gaz inerte ou dans la partie inférieure du condenseur à fractionnement.

3.  Procédé selon la revendication 1 ou 2, caractérisé en ce qu'on fait passer le courant de gaz inerte en circuit fermé.

4.  Procédé selon la revendication 1, caractérisé en ce qu'on utilise, au lieu du condenseur à fractionnement, un déflégmateur ou un colonne de distillation.

5.  Procédé selon l'une des revendications 1 à 3, caractérisé en ce qu'on utilise comme gaz inerte de l'azote, de l'air ou de l'air appauvri en oxygène, de l'anhydride carbonique ou un gaz rare.

6.  Procédé selon l'une des revendications 1 à 5, caractérisé en ce qu'on soumet à la séparation par distillation un mélange azéotrope de trioxanne et d'eau ou une solution aqueuse de trioxanne et de formaldéhyde.

7.  Procédé selon l'une des revendications 1 à 5, caractérisé en ce qu'on soumet à la séparation par distillation un mélange acide contenant du trioxanne, de l'eau et du formaldéhyde.

8.  Procédé selon l'une des revendications 1 à 5, caractérisé en ce qu'on soumet à la séparation par distillation un mélange acide contenant du trioxanne, de l'eau et du formaldéhyde et on exécute le procédé de séparation par distillation en présence d'un courant de gaz inerte contenant du formaldéhyde.

9.  Procédé selon l'une des revendications 1 à 4, caractérisé en ce qu'on soumet à la séparation par distillation un mélange acide contenant du trioxanne, de l'eau et du formaldéhyde et on utilise comme courant de gaz inerte contenant du formaldéhyde un courant gazeux de formaldéhyde issu de l'oxydation catalytique de méthanol.

10.  Procédé selon l'une des revendications 1, 6-9, caractérisé en ce qu'on effectue la séparation par distillation en discontinu, la teneur en eau, condensée dans le condenseur à fractionnement, du mélange de vapeur s'évacuant par distillation de l'évaporateur étant recyclée dans le système.

11.  Procédé selon l'une des revendications 1, 6 et 7, caractérisé en ce qu'on effectue la séparation par distillation en continu, la teneur en eau, condensée dans le condenseur à fractionnement, du mélange de vapeur s'évacuant par distillation de l'évaporateur étant éliminée du système.